# EUROPEAN PATENT APPLICATION

(11) **EP 2 862 922 A1**
(43) Date of publication of application: **22.04.2015**
(21) Application number: 12879294.2
(22) Date of filing: 19.06.2012
(51) Int. Cl.: C12M 1/00, C12N 15/09

(54) **CELL CULTURING VESSEL, AND CELL CULTURING METHOD AND AUTOMATED CELL CULTURING DEVICE USING SAME**

(71) Applicant: Hitachi, Ltd., Tokyo 100-8280 (JP)
(72) Inventor: ZHOU Guangbin, Kokubunji-shi Tokyo 185-8601 (JP); NOZAKI Takayuki, Kokubunji-shi Tokyo 185-8601 (JP); MATSUOKA Shizu, Kokubunji-shi Tokyo 185-8601 (JP)
(74) Representative: MERH-IP Matias Erny Reichl Hoffmann
(86) International application number: PCT/JP2012/065572
(87) International publication number: WO 2013/190630

(57) **Abstract**

The purpose of the present invention is to decrease cell culturing vessel running cost and reduce the amount of industrial waste. A cell culturing vessel provided with a container frame and a bottom face support wherein: the cell culturing space is formed by the frame and the bottom face support; the frame is provided with a securing means for securing the bottom face support so as to be attachable and detachable; and an exchangeable culturing surface is provided on the bottom face support.

## Description

### Technical Field

The present invention relates to a cell culturing vessel and a cell culturing method using the cell culturing vessel. Background

Conventionally, successive culturing of various types of cells (floating cell, adhesive cell) has been conducted. Especially, the adhesion dependent cell is proliferated while being in contact with the bottom face (culturing surface) of the culturing vessel. It is therefore necessary to pay attention to constantly keep the cell density within a specified range. If the cell density becomes so low that the distance between adjacent cells is increased, the adhesion dependent cell may become extinct owing to decreased proliferation rate. On the contrary, if the cell density becomes excessively high to reduce the distance between the adjacent cells, the adhesion dependent cell stops the cell proliferation, resulting in extinction. Upon culturing of the adhesion dependent cell, the successive cultivation is required to succeed the culturing so as to be performed in the subsequent culturing vessel.

In the cell culturing process, culturing is performed in the culturing vessel with small culturing area in an initial culturing stage so that the density of the adhesion dependent cell is not excessively lowered. At the timing when the adhesion dependent cell has been proliferated to a certain degree, culturing is succeeded to the culturing vessel with larger area or a plurality of culturing vessels each with the same culturing area. The aforementioned process is repeatedly executed to proliferate the adhesion dependent cells until the number of cells reaches the target value. Subsequent to the end of the cell culturing process, the culturing vessel is disposed as the medical industrial waste. Reuse of the culturing vessel requires cleaning, sterilization and surface treatment. Therefore, the culturing vessel is basically disposable, causing such problems as increased running cost and increased amount of industrial waste. Especially the main body of the culturing vessel with large area is manufactured through the significantly complicated process, thus further increasing the cost.

Patent Literature 1 proposes the cell culturing vessel configured to deposit the synthetic resin sheet to the culturing vessel frame. Patent Literature 2 proposes the cell culturing substrate configured to form the cell pattern using the film for transfer onto the desired target material.

### Related Art

### Patent Literature

Patent Literature 1: JP-A-2009-136156
Patent Literature 2: JP-A-2007-312736

### Summary of the Invention

### Problem to be Solved by the Invention

The cell culturing vessel as disclosed both in Patent Literatures 1 and 2 is not configured to allow only the culturing surface to be exchangeable.

An object of the present invention is to provide the culturing vessel with the cell culturing surface exchangeable so as to facilitate reduction in the running cost of the cell culturing vessel and in the amount of the industrial waste.

### Means for Solving the Problem

As an example of the cell culturing vessel according to the present invention, the cell culturing vessel including a container frame and a bottom face support is configured to form a cell culturing space by the frame and the bottom face support. The frame is provided with a securing member for detachably securing the bottom face support. An exchangeable culturing surface is provided on the bottom face support.

As another example of the cell culturing vessel according to the present invention, a cell culturing vessel with a frame for forming a cell culturing space with a bottom face, a side face and an upper face includes a culturing surface disposed on the bottom face of the cell culturing space, a cell peeling mechanism disposed on the culturing surface, and a mechanism of continuously exchanging the culturing surface.

The present invention provides a cell culturing method using the above-described cell culturing vessel, which includes steps of performing a cell culturing using the cell culturing vessel, exchanging the culturing surface after finishing the cell culturing, and sterilizing components except the culturing surface.

The automated cell culturing device according to the present invention includes the above-described cell culturing vessel, a culture medium supply mechanism for supplying a culture medium into the culturing vessel, a mixture gas supply mechanism for supplying the mixture gas into the culturing vessel, and a cell observation mechanism for observing the culturing surface of the culturing vessel.

### Effect of the Invention

The present invention allows reduction in the running cost of the cell culturing vessel and in the amount of the industrial waste.

### Brief Description of Drawing

Fig. 1 is an assembly diagram illustrating a cell culturing vessel according to a first embodiment of the present invention.
Fig. 2 is an exploded view of the cell culturing vessel according to the first embodiment of the present invention.
Fig. 3 is a view showing an exemplary structure of the culturing surface with an O-ring according to the present invention.
Fig. 4 is a view representing a structure for adsorption-desorption of the film culturing surface.
Fig. 5 is a view representing a method of adsorption-desorption of the film culturing surface.
Fig. 6 is an assembly diagram illustrating a cell culturing vessel of close type according to a second embodiment of the present invention.
Fig. 7 is an exploded view of the cell culturing vessel of close type according to the second embodiment of the present invention.
Fig. 8 is an assembly diagram illustrating another example of the cell culturing vessel with continuous culturing surface exchange structure according to the second embodiment of the present invention.
Fig. 9 is an exploded view of another example of the cell culturing vessel with continuous culturing surface exchange structure according to the second embodiment of the present invention.
Fig. 10 is a sectional view of the cell culturing vessel with continuous culturing surface exchange structure according to a third embodiment of the present invention.
Fig. 11 is a plan view of the cell culturing vessel with continuous culturing surface exchange structure according to the third embodiment of the present invention.
Fig. 12 is a view representing a method of culturing performed in the cell culturing vessel with continuous culturing surface exchange structure according to the third embodiment of the present invention.
Fig. 13 is a sectional view illustrating a cell culturing vessel with 2-layer continuous culturing surface exchange structure according to a fourth embodiment of the present invention.

### Embodiments for Carrying out the Invention

Embodiments for carrying out the present invention will be described referring to the drawings. In all the drawings for explaining embodiments for carrying out the invention, the element with the same function will be designated with the same term and code, and repetitive explanations thereof, thus will be omitted.

### First Embodiment

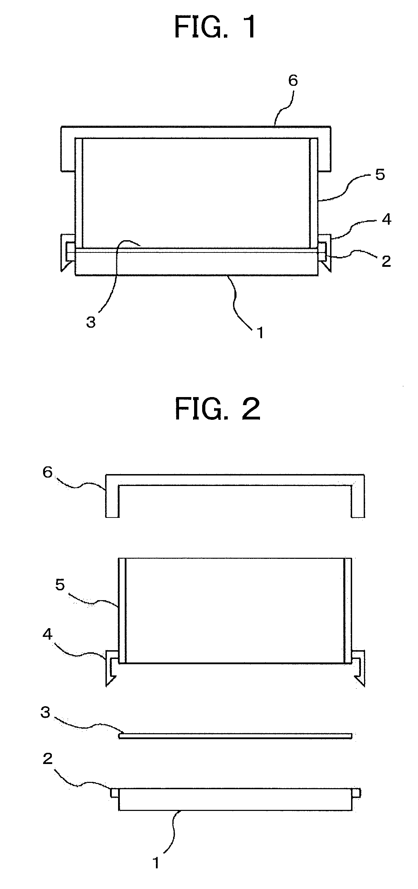

A cell culturing vessel according to a first embodiment of the present invention will be described based on Figs. 1 and 2. Fig. 1 is an assembly diagram of the cell culturing vessel of the first embodiment, and Fig. 2 is an exploded view of the assembly.

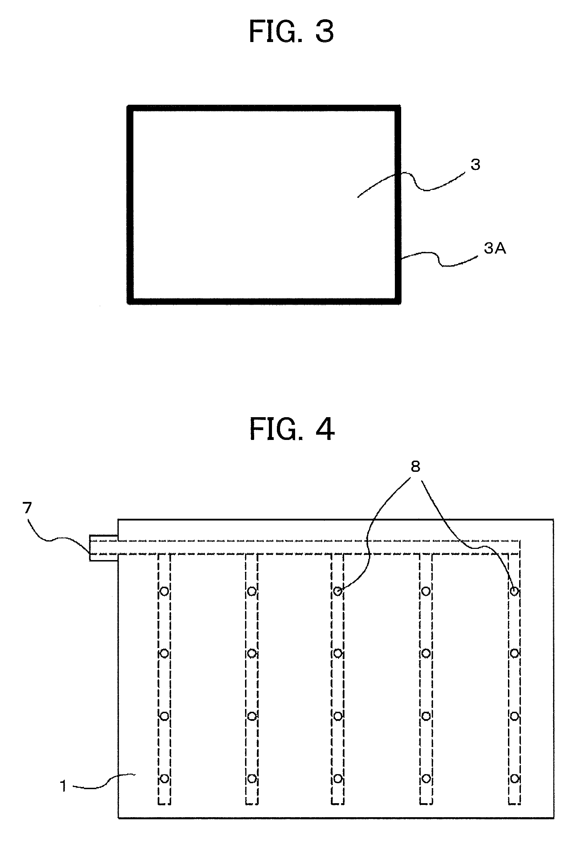

Referring to Figs. 1 and 2, a numeral 1 denotes a bottom face support that constitutes the bottom face of a cell culturing space, 2 denotes a protrusion attached to the bottom face support, 3 denotes a culturing surface, 4 denotes a lock member attached to a frame, 5 denotes a cylindrical frame that constitutes the side surface of the cell culturing space, and 6 denotes a lid that covers the frame. The culturing surface 3 is set on the bottom face support 1, on which the frame 5 is mounted. The frame 5 and the bottom face support 1 are detachably secured with the lock member 4 and the protrusion 2 which are formed on those components, respectively. The lid 6 is further disposed to complete assembly of the culturing vessel. The culturing surface 3 is exchangeable, and may be made of the film, the sheet or the plate which has been subjected to the cell adsorption process. The seal member is disposed as necessary to improve the sealing property between the frame 5 and the bottom face support 1. An O-ring 3A structure with good sealing property is fitted with the outer periphery of the culturing surface 3 as the seal member as shown in Fig. 3. The O-ring 3A may be formed separately from the culturing surface 3. Alternatively, it may be fixed to the culturing surface 3 through adhesion so as to be integrally formed. The O-ring is integrally formed with the culturing surface preliminarily to allow the culturing surface to be easily exchanged.

The flow of the cell culturing process using the cell culturing vessel of the embodiment will be described. Firstly, suspension of the target cell is injected into the culturing vessel for the target cell culturing. The culturing vessel is put into the incubator for the cell culturing so as to perform the cell culturing. The culture medium in the culturing region is discharged approximately at the timing of the cell proliferation limit on the culturing surface. Then the cell is washed in PBS (physiological salt water). Subsequently, trypsin is injected into the initial culturing region to peel the cell from the culturing surface so that the culture medium is injected to allow the cell to float therein. Thereafter, the cell suspension which contains the cell is collected. Conventionally, upon completion of the cell culturing process, the used culturing vessel is sterilized with the autoclave so as to be disposed of as the industrial waste. On the contrary, in the case of the culturing vessel according to the present invention, the lock member 4 and the protrusion 2 are detached from each other after collection of the cell suspension to only exchange the used culturing surface 3 with the new one. The other components are sterilized for reuse. This makes it possible to facilitate reduction in the running cost of the cell culturing vessel and in the amount of industrial waste.

It is preferable to use the material suitable for the cell culturing as the member for forming the exchangeable culturing surface 3, for example, poly-carbon, polystyrene and the like. It is preferable to use the soft material such as silicon for forming the O-ring 3A as the seal member.

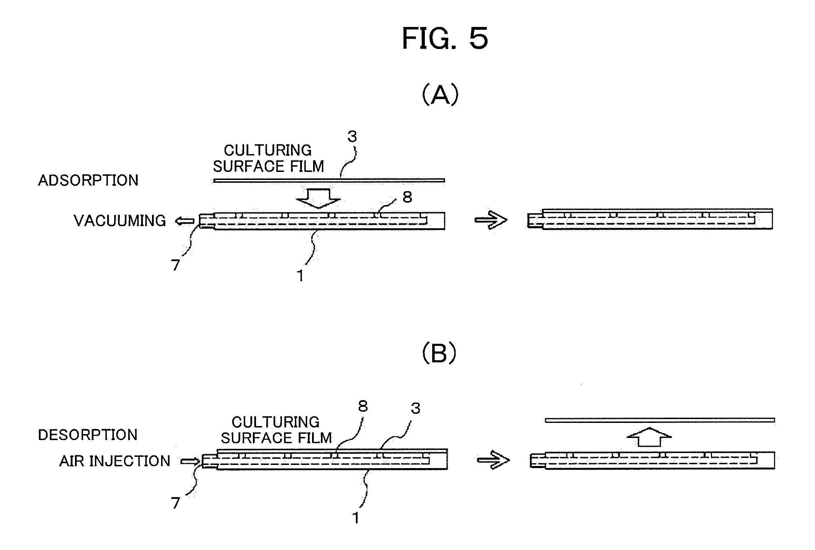

In the description, the culturing surface 3 is gripped and fixed between the frame 5 and the bottom face support 1. However, the method of fixing the culturing surface 3 is available through the vacuuming structure formed in the bottom face support 1 as shown in Fig. 4. Referring to Fig. 4, a numeral 7 denotes a vacuuming port, and 8 denotes a vacuuming hole. Fig. 5 represents the process of adsorbing and desorbing the culturing surface 3. As Fig. 5(a) shows, air is discharged through the vacuuming port 7 of the bottom face support 1 so that the culturing surface film 3 is adsorbed through the vacuuming holes 8 so that the culturing surface film 3 may be fixed to the surface of the bottom face support 1 in tight adherence under the atmospheric pressure. On the contrary, as Fig. 5(b) shows, upon introduction of air from the vacuuming port 7, air is fed from the vacuuming holes 8 so that the fixed culturing film 3 is desorbed from the surface of the bottom face support 1 by the action of air. This process allows the film type culturing surface to be adhesively fixed to the support face without using especially the adhesive at the constant pressure with further tight adherence.

It is possible to produce the lid 6 and the bottom face support 1 of the aforementioned culturing vessel by using the solid substrate as the base material, for example, glass, silicon, quarts, or plastics and polymer. More preferably, such material exhibits light transmission sufficient to observe with the optical microscope. Further preferably, the culturing surface 3 uses the material which allows the surface modification of the substrate through cleaning and preprocessing before adhesion of the cell to the surface.

It is needless to say that the outer frame of the culturing vessel may have the circular shape or the polygonal shape such as the rectangular shape.

The embodiment allows reduction in the running cost of the cell culturing vessel and in the amount of industrial waste.

### Second Embodiment

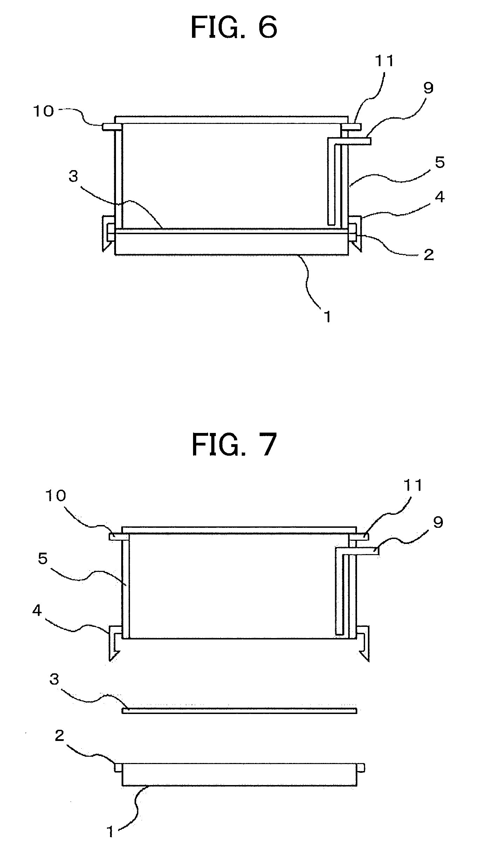

The cell culturing vessel of close type according to a second embodiment will be described referring to Figs. 6 and 7. Fig. 6 is an assembly diagram, and Fig. 7 is an exploded view of the assembly according to the second embodiment.

Figs. 6 and 7 show the similar vessel to that of the first embodiment except a culture medium inlet-outlet 9, a mixture gas inlet 10, and a mixture gas outlet 11.

The culturing vessel according to the embodiment is configured to form the cell culturing space by the frame 5 and the bottom face support 1, and includes the inlet 10 and the outlet 11 for the culturing mixture gas in the culturing space, the inlet-outlet 9 for exchanging the culture medium in the culturing space, and securing members including the lock member 4 and the protrusion 2 which are attached to the frame 5 and the bottom face support 1, respectively as well as the exchangeable culturing surface 3 disposed on the bottom face support 1. Preferably, air containing 5% of CO₂ at humidity of 90% or higher is used as the mixture gas.

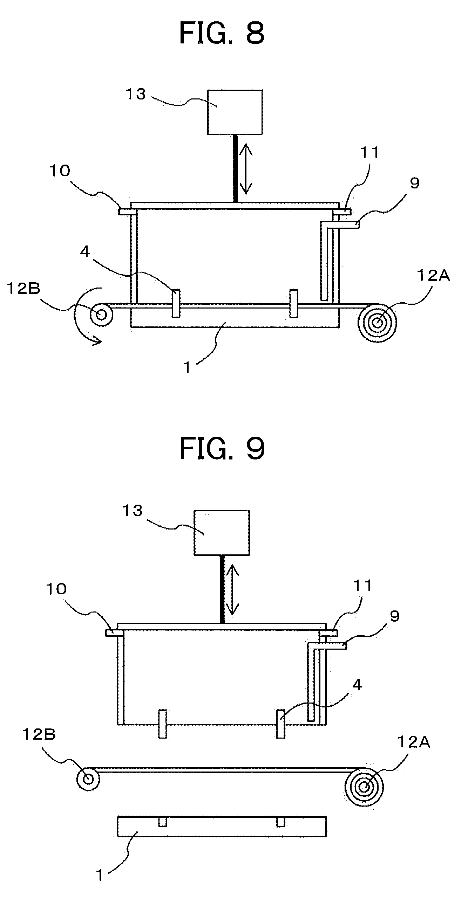

The culturing surface 3 according to the embodiment is manually exchanged. The structure as shown in Figs. 8 and 9 allows automated exchange of the culturing surface. The structure shown in Figs. 8 and 9 is basically the same as the one shown in Figs. 6 and 7 except a film supply roll 12A, a film collection roll 12B, and a lift mechanism 13. Upon exchange of the culturing surface 3, the lift mechanism 13 moves the frame 5 up for detachment from the bottom face support 1. Then the film collection roll 12B is wound so that the new culturing surface is supplied into the culturing vessel from the film supply roll 12A. Finally, the lift mechanism 13 moves the frame 5 down for attachment to the bottom face support 1 with the lock member 4 and the protrusion 2. In this way, the automated exchange of the culturing surface 3 is finished.

The aforementioned cell culturing film of roll type allows easy preprocessing, and is suitable for mass processing. It is possible to form the cell culturing pattern on the film so that culturing of various types of cell tissue, for example, the cell sheet culturing may be performed.

The culturing vessel of the embodiment supplies the mixture gas and the culture medium directly to the culturing space to allow the automated culturing in the close system. This embodiment is capable of facilitating reduction in the running cost of the cell culturing vessel and in the amount of industrial waste.

### Third Embodiment

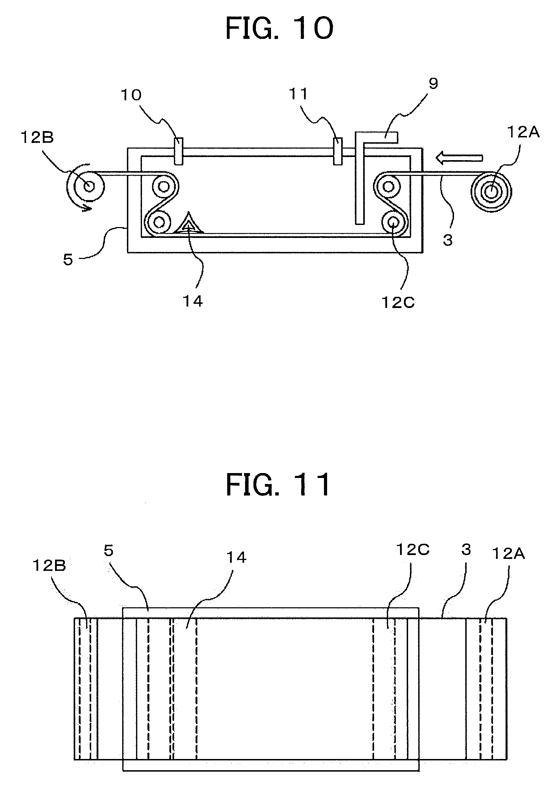

Described below is the cell culturing vessel with the structure of continuously exchangeable culturing surface according to a third embodiment of the present invention referring to Figs. 10 and 11. Fig. 10 is a sectional view, and Fig. 11 is a plan view of the cell culturing vessel according to the third embodiment.

The culturing vessel of the embodiment is configured to form the cell culturing space by the frame 5 of the culturing vessel, and includes the inlet 10 and the outlet 11 for the culturing mixture gas in the culturing space, the inlet-outlet 9 for exchanging the culture medium in the culturing space, a cell peeling mechanism 14 formed on the culturing surface 3, and a structure that allows continuous exchange of the culturing surface 3. In this embodiment, the culturing surface 3 is made of the film. The film as the culturing surface is supplied from the film supply roll 12A into the culturing vessel so as to be disposed on the bottom face of the culturing vessel with the rotating roll 12C. Upon exchange of the film, the film is wound by the film collection roll. The cell peeling mechanism 14 is disposed on the surface of the film near the rotating roll 12C at the film collection side. The frame 5 of the culturing vessel includes the inlet 10 and the outlet 11 for the culturing mixture gas, and the culture medium exchange inlet-outlet 9 in the culturing space.

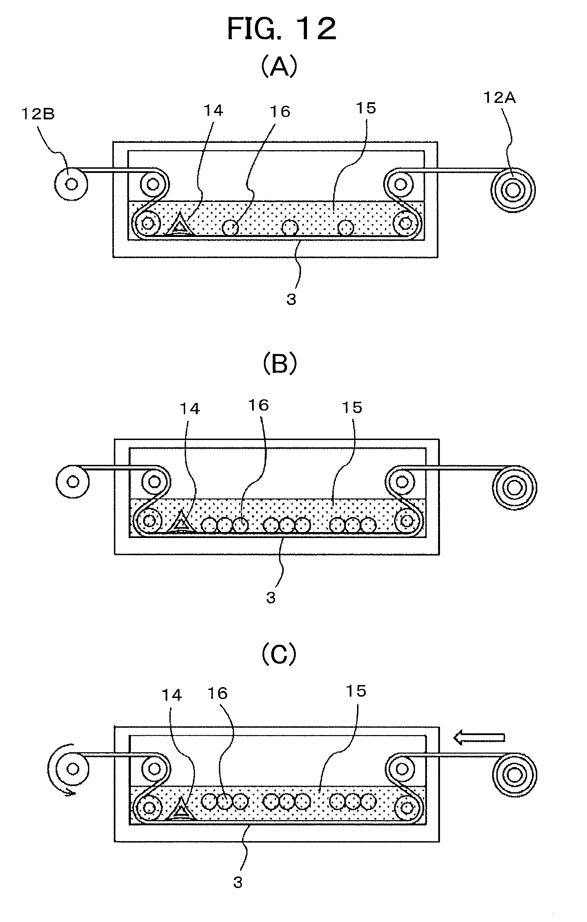

The culturing process will be described referring to Fig. 12. A reference numeral 15 denotes the culture medium, and 16 denotes the cell. As Fig. 12(a) shows, the culture medium 15 is introduced onto the culturing surface 3 in the culturing vessel to seed a target cell 16 for culturing. The culturing is stopped approximately at the timing of the cell proliferation limit on the culturing surface (Fig. 12(b)). As Fig. 12(c) shows, the cell 16 on the culturing surface is peeled off using the cell peeling mechanism 14, for example, the scraper while winding the film collection roll 12B so that the peeled cell is suspended in the culture medium 15. After collecting the cell suspension, the cell can be seeded on the new culturing surface again for successive cultivation. The embodiment allows reduction in the running cost of the cell culturing vessel, and in the amount of industrial waste.

The culturing vessel according to the embodiment may be used for successive cultivation of iPS (induced pluripotent stem cell).

It is also possible to produce the automated cell culturing device by the cell culturing vessel according to the embodiment, the culture medium supply mechanism for supplying the culture medium into the culturing vessel, the mixture gas supply mechanism for supplying the mixture gas into the culturing vessel, and the cell observation mechanism for observing the culturing surface in the culturing vessel.

### Fourth Embodiment

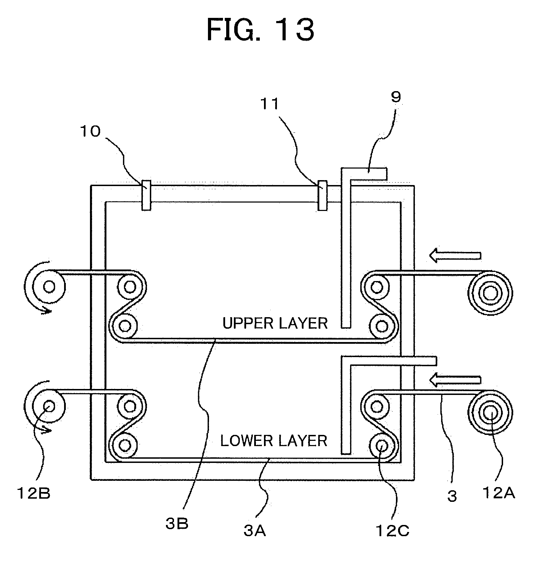

A cell culturing vessel with a 2-layer continuous culturing surface exchange structure will be described referring to Fig. 13.

The structure shown in Fig. 13 is similar to the one described in the third embodiment except the 2-layer culturing surface.

The culturing vessel according to the embodiment is configured to form the cell culturing space by the frame of the culturing vessel, the inlet 10 and the outlet 11 for the culturing mixture gas in the culturing space, the inlet-outlet 9 for exchanging the culture medium in the culturing space, and culturing surfaces 3A and 3B which allow 2-layer continuous exchange in the culturing space. Each of the culturing surfaces 3A and 3B is made of the film, and supplied into the culturing vessel from the film supply roll 12A. They are disposed on the bottom face of the culturing vessel by the rotating roll 12C. Upon exchange of the film, the film is wound by the film collection roll 12B.

The use of the porous film as the upper layer for the culturing vessel according to the embodiment allows co-cultivation of two or more kinds of cells likewise the insert type culturing vessel. The embodiment ensures to facilitate reduction in the running cost of the cell culturing vessel, and in the amount of industrial waste.

It is to be understood that the present invention is not limited to the aforementioned embodiments, and any possible embodiments may also be contained in the scope of the present invention without impairing characteristics of the present invention.

### Description of Code

- 1: bottom face support
- 2: protrusion
- 3: culturing surface
- 3A: O-ring
- 4: lock member
- 5: frame
- 6: lid
- 7: vacuuming port
- 8: vacuuming hole
- 9: culture medium inlet-outlet
- 10: mixture gas inlet
- 11: mixture gas outlet
- 12A: film supply roll
- 12B: film collection roll
- 12C: rotating roll
- 13: lift mechanism
- 14: cell peeling mechanism
- 15: culture medium
- 16: cell

## Claims

1. A cell culturing vessel including a container frame and a bottom face support, wherein:
a cell culturing space is formed by the frame and the bottom face support;
the frame is provided with a securing member for detachably securing the bottom face support; and
an exchangeable culturing surface is provided on the bottom face support.

2. The cell culturing vessel according to claim 1, wherein the frame forms a cylindrical side surface of the cell culturing space, and a lid is provided for covering the frame.

3. The cell culturing vessel according to claim 1, wherein the frame forms a cylindrical side surface of the cell culturing space and a lid.

4. The cell culturing vessel according to claim 3, further comprising:
an inlet for introducing culturing mixture gas into the cell culturing space and an outlet for discharging the culturing mixture gas; and
a culture medium exchange inlet-outlet for introducing or discharging a culture medium into or from the cell culturing space.

5. The cell culturing vessel according to any one of claims 1 to 4, wherein the exchangeable culturing surface is made of a film, sheet or a plate which has been subjected to a cell adsorption process.

6. The cell culturing vessel according to any one of claims 1 to 5, wherein the securing member includes a protrusion disposed on one of the bottom face support and the frame, and a lock member disposed on the other one.

7. The cell culturing vessel according to any one of claims 1 to 6, wherein a seal member is interposed between the exchangeable culturing surface and the frame.

8. The cell culturing vessel according to claim 7, wherein the seal member is integrally formed with the culturing surface preliminarily.

9. The cell culturing vessel according to any one of claims 1 to 8, wherein the bottom face support includes a structure of adsorbing and desorbing the culturing surface.

10. The cell culturing vessel according to claim 3, further comprising a lift mechanism for moving the frame up and down, wherein a continuously exchangeable culturing surface is disposed on the bottom face support.

11. A cell culturing vessel with a frame for forming a cell culturing space with a bottom face, a side face and an upper face, comprising:
a culturing surface disposed on the bottom face of the cell culturing space;
a cell peeling mechanism disposed on the culturing surface; and
a mechanism of continuously exchanging the culturing surface.

12. The cell culturing vessel according to claim 11, wherein:
the culturing surface is made of a continuous film or a sheet which has been subjected to a cell adsorption process; and
the mechanism of continuously exchanging the culturing surface includes a supply roll for supplying the film or the sheet, and a collection roll for collecting the film.

13. The cell culturing vessel according to claim 11 or 12, wherein the culturing space is provided with a 2-layer continuously exchangeable culturing surface.

14. A cell culturing method using the cell culturing vessel according to any one of claims 1 to 13, comprising:
step of performing a cell culturing using the cell culturing vessel;
step of exchanging the culturing surface after finishing the cell culturing; and
step of sterilizing components except the culturing surface.

15. An automated cell culturing device comprising:
the cell culturing vessel according to any one of claims 1 to 13;
a culture medium supply mechanism for supplying a culture medium into the culturing vessel;
a mixture gas supply mechanism for supplying the mixture gas into the culturing vessel; and
a cell observation mechanism for observing the culturing surface of the culturing vessel.
